# EUROPEAN PATENT APPLICATION

(11) **EP 4 672 257 A1**
(43) Date of publication of application: **31.12.2025**
(21) Application number: 25185356.0
(22) Date of filing: 25.06.2025
(51) Int. Cl.: G16H 30/20, G16H 30/40, G16H 50/20

(54) **INFORMATION PROCESSING DEVICE AND OPERATION METHOD OF INFORMATION PROCESSING DEVICE**

(30) Priority: 26.06.2024 JP 2024102629
(71) Applicant: FUJIFILM Corporation, Tokyo 106-8620 (JP)
(72) Inventor: YUZAWA, Takuya, Tokyo, 1068620 (JP)
(74) Representative: Dehns Germany Partnerschaft mbB

(57) **Abstract**

An aspect of the present invention provides an information processing device and an operation method of an information processing device, which can appropriately store a medical image. An information processing device according to an aspect of the present invention is an information processing device including a processor, in which the processor is configured to: acquire medical data including at least one of a medical image or medical comments on findings related to the medical image; apply a machine learning method to the medical data to detect predetermined medical information from the medical data; and move the medical image stored in a transitory storage region of a storage device to a long-term storage region of the storage device in a case where the medical information is detected.

## Description

### BACKGROUND OF THE INVENTION

### 1. Field of the Invention

The present invention relates to an information processing device and an operation method of an information processing device, and particularly relates to a technique for storing medical information.

### 2. Description of the Related Art

Regarding the technique for storing medical information, for example, JP2015-228921A discloses that temporarily stored data is stored in a permanent storage device in response to a user instruction.

### SUMMARY OF THE INVENTION

As a system such as a picture archiving and communication system (PACS), a system comprising a long-term storage region in which data such as a medical image is stored for a long period of time and a transitory storage region in which data is automatically deleted according to a lapse of a predetermined period of time, a remaining capacity of a storage, and the like is known. A data size of the medical image may be large depending on imaging conditions such as a modality and a slice thickness, and in a case where such a medical image is always stored in the long-term storage region, a storage capacity is strained. Therefore, the medical image is stored in the transitory storage region.

In addition, in recent years, development of a CT device (photon counting CT (PCCT), CT is computer tomography) comprising a photon counting type detector has been promoted. In the PCCT device, a higher resolution image can be obtained as compared with a CT device in the related art, and an image (material discrimination image) in which materials having different attenuation coefficients of radiation are discriminated and visualized can be obtained. Since a data size of these high-resolution images and material discrimination images is extremely large and the storage is strained, an operation of storing the high-resolution images and the material discrimination images in the transitory storage region is assumed.

In a case where a user uses an image in the transitory storage region for diagnosis, for example, the user may want to store the image in the long-term storage region and refer to the image thereafter. In addition, in a case where the user registers the image in the transitory storage region in a report system as a key image or bookmarks the image during interpretation of the image in a viewer, the image in the transitory storage region is copied or moved to the long-term storage region.

However, since the user does not always register the key image or perform bookmarking, in a case where such an operation is not performed, a situation in which "the image has already been deleted because it was stored in the transitory storage region, and thus the image cannot be referred to even though the image is desired to be referred to" may occur. However, the techniques in the related art such as JP2015-228921A described above have not taken these problems into consideration.

The present invention has been made in view of such circumstances, and provides an information processing device and an operation method of an information processing device, which can appropriately store a medical image.

An information processing device according to a first aspect of the present invention is an information processing device comprising a processor, in which the processor is configured to: acquire medical data including at least one of a medical image or medical comments on findings related to the medical image; apply a machine learning method to the medical data to detect predetermined medical information from the medical data; and move the medical image stored in a transitory storage region of a storage device to a long-term storage region of the storage device in a case where the medical information is detected.

According to the first aspect, in a case where the predetermined medical information is detected, the medical image stored in the transitory storage region of the storage device can be automatically moved to the long-term storage region of the storage device. Therefore, it is possible to reduce a possibility of a situation in which "the medical image stored in the transitory storage region has been deleted, and thus the image cannot be referred to even though the image is desired to be referred to", and it is possible to appropriately store the medical image. The "predetermined medical information" may be, for example, information important for diagnosis or report creation.

In the first aspect and each of the following aspects, it is assumed that the term "automatically" means that, in a case where a determined condition is satisfied, a determined process or operation (in the first aspect, movement of medical information) is executed regardless of an operation of a user. As a result, even for a medical image for which the user has not registered as the key image or performed bookmarking, in a case where the predetermined medical information is detected, the medical image can be moved to the long-term storage region, and it is possible to reduce a burden on the user regarding the storage of the medical image.

In the first aspect and each of the following aspects, the medical data includes at least one of the medical image or the medical comments on findings related to the medical image. Therefore, the processor may detect the "predetermined medical information" from one of the medical image or the medical comments on findings, or may detect the "predetermined medical information" from both the medical image and the medical comments on findings. It is preferable that the medical image and the medical comments on findings are linked to each other (associated with each other).

In addition, in the first aspect and each of the following aspects, the machine learning method used for detecting the "predetermined medical information" is not particularly limited. In addition to deep learning such as a convolutional neural network (CNN), a recurrent neural network (RNN), long short-term memory (LSTM), and Attention, algorithms such as a decision tree, a random forest, a support vector machine, and a naive Bayes may be used.

In the first aspect and each of the following aspects, a storage period of time of the long-term storage region and the transitory storage region is not particularly limited, but it is assumed that the long-term storage region is a region in which information is stored for a longer period of time than the transitory storage region. The long-term storage region may be a permanent storage region (a storage region in which information is not deleted unless the user performs an operation).

In an information processing device according to a second aspect of the present invention, in the first aspect, the processor is configured to: restore the medical image moved to the long-term storage region to the transitory storage region in a case where the processor acquires information indicating that a result of the detection is erroneous detection. The second aspect takes into account that there is a low necessity to store the medical image in the long-term storage region in a case of the erroneous detection. In the second aspect and each of the following aspects, the processor may acquire the "information indicating the erroneous detection" based on a processing result of the processor or another information processing device, or may acquire information input by a user who performs interpretation of medical images, and the like as the "information indicating the erroneous detection".

In an information processing device according to a third aspect, in the first or second aspect, the processor is configured to: move the medical image from the transitory storage region to the long-term storage region in a case where the processor acquires information indicating that the medical image is displayed on a display device together with a result of the detection (result of the detection of the "predetermined medical information" defined in the first aspect). The processor can determine that the "predetermined medical information" in the first aspect is detected in a case where the processor acquires the information defined in the third aspect.

In an information processing device according to a fourth aspect, in any one of the first to third aspects, the processor is configured to: move a medical image related to specific information from the transitory storage region to the long-term storage region in a case where the specific information is detected from the medical comments on findings.

In an information processing device according to a fifth aspect, in the fourth aspect, the processor is configured to: detect information indicating an organ which is a target of the medical comments on findings as the specific information from the medical comments on findings and move a medical image in which the organ is shown from the transitory storage region to the long-term storage region. The fifth aspect defines a specific aspect of the "specific information" in the fourth aspect.

In an information processing device according to a sixth aspect, in the fourth or fifth aspect, the processor is configured to: detect a specific phrase as the specific information from the medical comments on findings and move a medical image related to the specific phrase.

In an information processing device according to a seventh aspect, in the sixth aspect, the processor is configured to: detect a phrase related to a region of interest as the specific phrase. The term "phrase related to a region of interest" may be, for example, one or more of a presence, type, size, shape, or color of the region of interest. The "region of interest" (ROI) is also referred to as a "region-of-attention".

In an information processing device according to an eighth aspect, in any one of the fourth to seventh aspects, the processor is configured to: detect the specific information from the medical comments on findings using a natural language processing method. The processor may perform natural language processing using the machine learning method. As described above for the first aspect, the machine learning method used in the eighth aspect is not particularly limited, and may be deep learning such as CNN, RNN, LSTM, and Attention, or may be a decision tree, a random forest, a support vector machine, a naive Bayes, or the like.

In an information processing device according to a ninth aspect, in any one of the first to eighth aspects, the processor is configured to: move a first medical image and a second medical image which is related to the first medical image and is different from the first medical image in a type of image, from the transitory storage region to the long-term storage region, in a case where a region of interest is detected in the first medical image.

In an information processing device according to a tenth aspect, in the ninth aspect, the second medical image is an image different from the first medical image in at least one of a modality, an imaging parameter, or a content of image processing.

In an information processing device according to an eleventh aspect, in the ninth or tenth aspect, the processor is configured to: move a medical image in which a designated organ and/or region of interest is shown, out of the second medical image.

In an information processing device according to a twelfth aspect, in any one of the first to eleventh aspects, the processor is configured to: acquire a CT image and/or a material discrimination image as the medical image.

In an information processing device according to a thirteenth aspect, in the twelfth aspect, the CT image and the material discrimination image are images captured by a photon counting CT device.

In an information processing device according to a fourteenth aspect, in any one of the seventh aspect, the ninth aspect, or the eleventh aspect, the region of interest is at least one of a lesion, a lesion candidate region, or a treated region.

In an information processing device according to a fifteenth aspect, in any one of the first to fourteenth aspects, the processor is configured to: cause an output device to output information indicating the medical data stored in the transitory storage region and/or information indicating the medical data stored in the long-term storage region.

In an information processing device according to a sixteenth aspect, in any one of the first to fifteenth aspects, the processor is configured to: cause an output device to output information indicating a history of movement of the medical image between the transitory storage region and the long-term storage region.

In an information processing device according to a seventeenth aspect, in any one of the first to sixteenth aspects, the processor is configured to: move the medical image to a region that stores the medical image for a longer period of time than the transitory storage region by using the region as the long-term storage region.

An operation method of an information processing device according to an eighteenth aspect is an operation method of an information processing device including a processor, the method comprising: via the processor, acquiring medical data including at least one of a medical image or medical comments on findings related to the medical image; applying a machine learning method to the medical data to detect predetermined medical information from the medical data; and moving the medical image stored in a transitory storage region of a storage device to a long-term storage region of the storage device in a case where the medical information is detected. According to the eighteenth aspect, it is possible to appropriately store the medical image as in the first aspect, and it is possible to reduce the burden on the user regarding the storage of the medical image. In addition, a program that causes a computer to execute the operation method according to the eighteenth aspect, a program product, and a non-transitory and tangible recording medium in which a computer-readable code of the program is recorded can also be given as aspects of the present invention. It is assumed that the "non-transitory and tangible recording medium" does not include a non-tangible recording medium such as a carrier wave signal or a propagation signal itself.

As described above, according to the information processing device and the operation method of an information processing device according to the aspect of the present invention, it is possible to appropriately store the medical image, and it is possible to reduce the burden on the user regarding the storage of the medical image.

### BRIEF DESCRIPTION OF THE DRAWINGS

Fig. 1 is a diagram showing a configuration of a medical information processing system.
Fig. 2 is a diagram showing a configuration of a main part of an information processing device.
Figs. 3A and 3B are schematic diagrams showing an example of a layer configuration of a recognizer.
Fig. 4 is a schematic diagram showing an example of a layer configuration of an interlayer.
Fig. 5 is a schematic diagram showing a configuration example of a detector by an RNN.
Fig. 6 is a flowchart showing processing in the medical information processing system.
Fig. 7 is a diagram showing an example of a movement history of a medical image.
Fig. 8 is a flowchart showing processing of restoring a medical image moved to a long-term storage region to a transitory storage region.
Fig. 9 is another flowchart showing processing in the medical information processing system.

### DESCRIPTION OF THE PREFERRED EMBODIMENTS

### First embodiment

### Configuration of medical information processing system

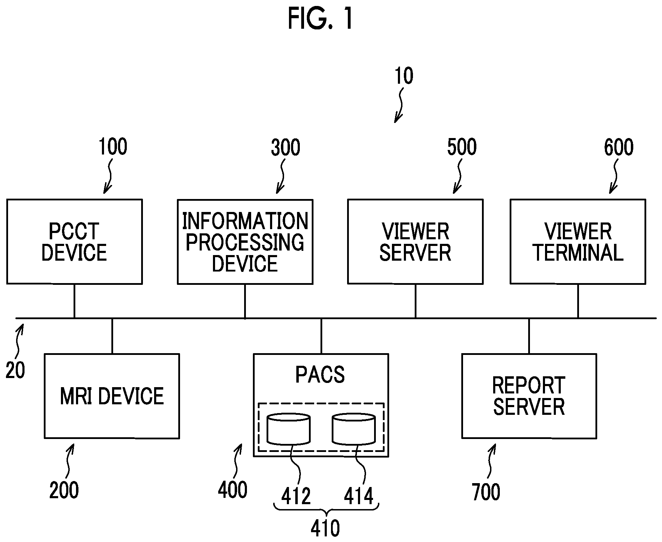

Fig. 1 is a diagram showing a configuration of a medical information processing system 10 (medical information processing system) according to a first embodiment. As shown in the drawing, the medical information processing system 10 comprises a photon counting CT (PCCT, CT is computer tomography) device 100, a magnetic resonance imaging (MRI) device 200, an information processing device 300 (information processing device), a picture archiving and communication system (PACS) 400, a viewer server 500, a viewer terminal 600, and a report server 700, and these devices are connected to a network 20 and are configured to communicate with each other as necessary.

The medical information processing system 10 may comprise another modality (medical image capturing device) such as a general CT device. The medical information processing system 10 may comprise a hospital information system (HIS), a radiology information system (RIS), and the like.

### PCCT device

A solid scintillation detector is mounted on a CT device in the related art. X-rays are converted into visible light by the solid scintillation detector, and the visible light is converted into an electric signal by photodiodes. On the other hand, a photon counting detector mounted on the PCCT device 100 can directly convert the X-ray photons into an electric signal. In the photon counting detector, the dose utilization efficiency is improved as compared with a detector in the related art, and since the pixel is extremely small, the spatial resolution can be significantly improved. As a result, in the PCCT device 100 (photon counting CT device), it is possible to obtain an image having high contrast and high resolution as compared with the CT device in the related art, and it is possible to reduce the radiation dose and the amount of contrast medium used.

The PCCT device 100 can capture and generate CT images (for example, slice positions, slice thicknesses, slice directions (axial, sagittal, coronal, and the like), highlighting of specific organs or regions of interest, and the like) having different imaging parameters and contents of image processing. In addition, the PCCT device 100 can generate not only a normal CT image but also an image (material discrimination image) in which materials having different attenuation coefficients of radiation are discriminated and visualized. It is preferable that the PCCT device 100 is a device capable of generating a plurality of material discrimination images having different contents and/or degrees of discrimination.

### MRI device

The MRI device 200 is a device that generates images of information from within a living body by using a nuclear magnetic resonance (NMR) phenomenon.

### PACS

The picture archiving and communication system (PACS) 400 is a system that receives medical images captured by modalities such as the PCCT device 100 and the MRI device 200 through the network 20 and stores and manages the medical images. The PACS is introduced in order to manage and share a large number of images, to meet the need for "immediate interpretation of a required image", and the like, and can improve the efficiency of the work of handling medical images by cooperating with other systems of medical institutions (including each element in Fig. 1).

The PACS 400 comprises a storage device 410 (storage device) that stores medical images and the like, and the storage device 410 is configured with a recording medium such as a magneto-optical recording medium and a semiconductor memory, and a control device thereof, and has a transitory storage region 412 (transitory storage region) and a long-term storage region 414 (long-term storage region). A storage period of time of the information in the transitory storage region 412 and the long-term storage region 414 is not particularly limited, but it is assumed that the long-term storage region 414 is a region in which information is stored for a longer period of time than the transitory storage region 412. The long-term storage region 414 may be a permanent storage region (a storage region in which information is not deleted unless the user performs an operation). The transitory storage region 412 and the long-term storage region 414 are preferably storage regions in a non-volatile recording medium. In addition, the transitory storage region 412 and the long-term storage region 414 may be different regions of a common recording medium, or may be each a storage region in a separate recording medium. The PACS 400 comprises a processor and a memory (which are not shown) in addition to the storage device 410.

In the first embodiment, the aspect in which the PACS 400 comprises the transitory storage region 412 and the long-term storage region 414 has been described, but instead of or in addition to this aspect, other devices such as the information processing device 300 may have the transitory storage region and the long-term storage region.

### Information processing device

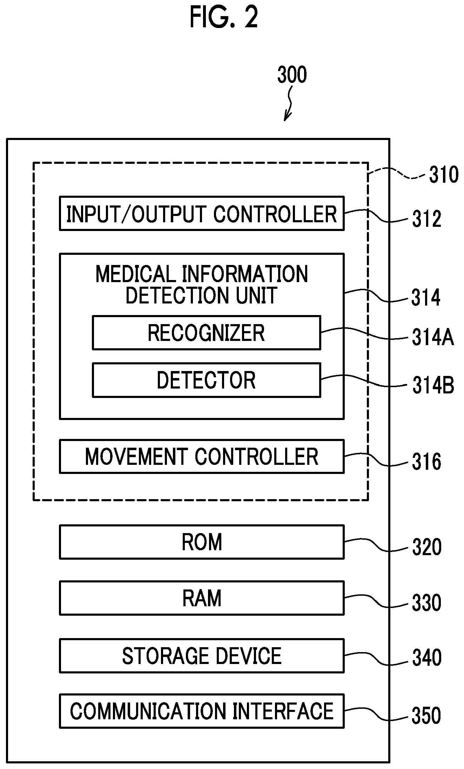

Fig. 2 is a diagram showing a main configuration of the information processing device 300 (information processing device). As shown in Fig. 2, the information processing device 300 comprises a processor 310 (processor), a read only memory (ROM) 320, a random access memory (RAM) 330, a storage device 340, and a communication interface 350.

### Processor

The processor 310 is configured by, for example, various processors, such as a central processing unit (CPU), a graphics processing unit (GPU), a field programmable gate array (FPGA), and a programmable logic device (PLD), and electric circuits. In a case where these processors and electric circuits execute software (program), codes readable by a computer (for example, various processors and electric circuits constituting the processor and/or combinations thereof) in the software to be executed are stored in a non-transitory and tangible recording medium, such as the ROM 320, and the computer refers to the software.

The software that is stored in the non-transitory and tangible recording medium includes the program according to the embodiments of the present invention (the operation method of an information processing device according to the embodiments of the present invention, and a program for operating the information processing device) and data used during the execution of the program. The code may be recorded on a non-transitory and tangible recording medium such as a flash ROM or an electronically erasable and programmable read only memory (EEPROM) instead of the ROM 320. The "non-transitory and tangible recording medium" does not include a non-tangible recording medium such as a carrier wave signal or a propagation signal itself. The RAM 330 is used as the transitory storage region or a work region during processing using software.

The processor 310 mainly comprises an input/output controller 312, a medical information detection unit 314, and a movement controller 316. The input/output controller 312 acquires medical data including at least one of a medical image or medical comments on findings related to the medical image from the storage device 410 of the PACS 400, the viewer server 500, or the report server 700, and acquires various processing conditions and outputs (stores and displays) processing results. The medical information detection unit 314 detects predetermined medical information from the acquired medical data. The movement controller 316 controls the movement of the medical image between the transitory storage region 412 and the long-term storage region 414. The processor 310 may have a configuration in which processing other than each unit described above is performed.

### Implementation of functions by various processors

The functions of each unit of the processor 310 described above can be implemented by using various processors and a recording medium. The various processors include, for example, a central processing unit (CPU) which is a general-purpose processor that implements various functions by executing software (programs). In addition, the various processors also include a graphics processing unit (GPU), which is a processor specialized for image processing, and a programmable logic device (PLD) which is a processor whose circuit configuration can be changed after manufacturing, such as a field programmable gate array (FPGA). In a case of performing learning and recognition of images as in the invention, a configuration using the GPU is effective. Further, the various processors described above also include a dedicated electric circuit, which is a processor having a circuit configuration specifically designed to execute specific processing such as an application specific integrated circuit (ASIC).

The functions of each unit may be implemented by one processor or may be implemented by a plurality of processors of the same type or different types (for example, a plurality of FPGAs, a combination of the CPU and the FPGA, or a combination of the CPU and the GPU). In addition, a plurality of functions may be implemented by one processor. A first example of the configuration in which a plurality of functions are configured by one processor is an aspect in which one processor is configured by a combination of one or more CPUs and software and implements a plurality of functions. A representative example of this aspect is a computer. A second example of the configuration is an aspect in which a processor that implements the functions of the entire system using one integrated circuit (IC) chip is used. A representative example of this aspect is a system-on-chip (SoC). As described above, various functions are configured using one or more of the above-described various processors as a hardware structure. In addition, specifically, an electric circuit (circuitry) obtained by combining circuit elements, such as semiconductor elements, can be used as the hardware structure of the various processors. The electric circuit may be an electric circuit that implements the above-described functions using a logical sum, a logical product, a logical negation, an exclusive logical sum, and a logical operation of a combination thereof.

In a case where the above-described processor or electric circuit is to execute software (program), codes, which can be read by a computer (for example, various processors and electric circuit constituting the processor 310, and/or a combination thereof) of the software to be executed are stored in a non-transitory recording medium such as the read only memory (ROM) 320, and the computer refers to the software. The software stored in the non-transitory recording medium includes programs for executing the operation method of an information processing device according to an embodiment of the present invention, and data used for execution (data regarding acquisition of medical data, data used for detecting medical information, parameters used in the recognizer or the detector described below, a list of specific phrases to be detected, and the like). The code or data may be recorded in the non-transitory recording medium, such as various magneto-optical recording devices and a semiconductor memory, instead of the ROM 320. In a case where processing is to be performed using software, for example, the random access memory (RAM) 330 is used as a transitory storage region, and the data stored in, for example, an electronically erasable and programmable read only memory (EEPROM) (not shown) can be referred to. The storage device 340 may be used as a non-transitory recording medium that records the code or the data.

The details of processing performed by each of these units will be described later.

### Construction of medical information detection unit by machine learning method

In the first embodiment, the medical information detection unit 314 comprises a recognizer 314A (processor) that recognizes a region of interest in a medical image (CT image, material discrimination image, MRI image, or the like), and a detector 314B (processor) that detects specific information from medical comments on findings. The recognizer 314A and the detector 314B can be constructed using a machine learning method.

### Configuration of recognizer

The recognizer 314A can be constructed by, for example, a neural network such as a convolutional neural network (CNN) or a deep neural network (DNN), a support vector machine (SVM), or the like, and can detect, discriminate, and measure a region of interest (ROI). The region of interest may be at least one of a lesion, a lesion candidate region, or a treated region. The region of interest may be referred to as a region-of-attention. Hereinafter, a layer configuration in a case where the recognizer is configured with the CNN will be described. Although the detector (recognizer that performs detection of the region of interest) will be mainly described, the same layer configuration can be employed for classification (discrimination) and measurement.

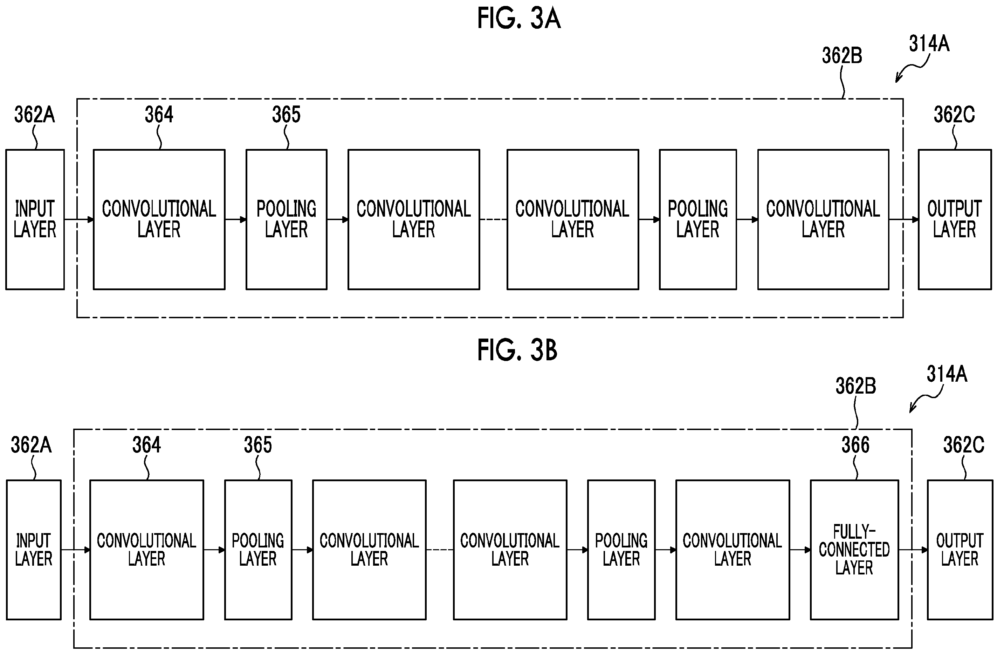

Figs. 3A and 3B are schematic diagrams showing an example of a layer configuration of the recognizer 314A. In the example shown in Fig. 3A, the recognizer 314A includes an input layer 362A, an interlayer 362B, and an output layer 362C. The input layer 362A receives the medical image acquired by the input/output controller 312 and outputs a feature amount. The interlayer 362B includes a convolutional layer 364 and a pooling layer 365, and the feature amount output by the input layer 362A is input to calculate other feature amounts. These layers have a structure in which a plurality of "nodes" are connected by "edges" and hold a plurality of weight parameters. The values of the weight parameters change as the learning progresses. As in the example shown in Fig. 3B, the recognizer 314A may include a fully-connected layer 366. The layer configuration of the recognizer 314A is not limited to a case in which the convolutional layer 364 and the pooling layer 365 are repeated one by one, and a plurality of layers (for example, the convolutional layer 364) may be continuously included. In addition, a plurality of fully-connected layers 366 may be continuously included.

### Processing in interlayer

The interlayer 362B calculates the feature amount by a convolution operation and a pooling process. The convolution operation performed in the convolutional layer 364 is processing of acquiring a feature map through convolution operations using filters, and plays a role of feature extraction such as edge extraction from the image. Through the convolution operations using filters, a "feature map" of one channel (one image) is generated for one filter. The size of the "feature map" is downscaled by convolution and becomes smaller as convolution is performed at each layer. The pooling process performed in the pooling layer 365 is processing of reducing (or enlarging) the feature map output by the convolution operation to create a new feature map and plays a role of providing robustness such that the extracted features are not affected by parallel translation or the like. The interlayer 362B can be composed of one or a plurality of layers that perform these processes.

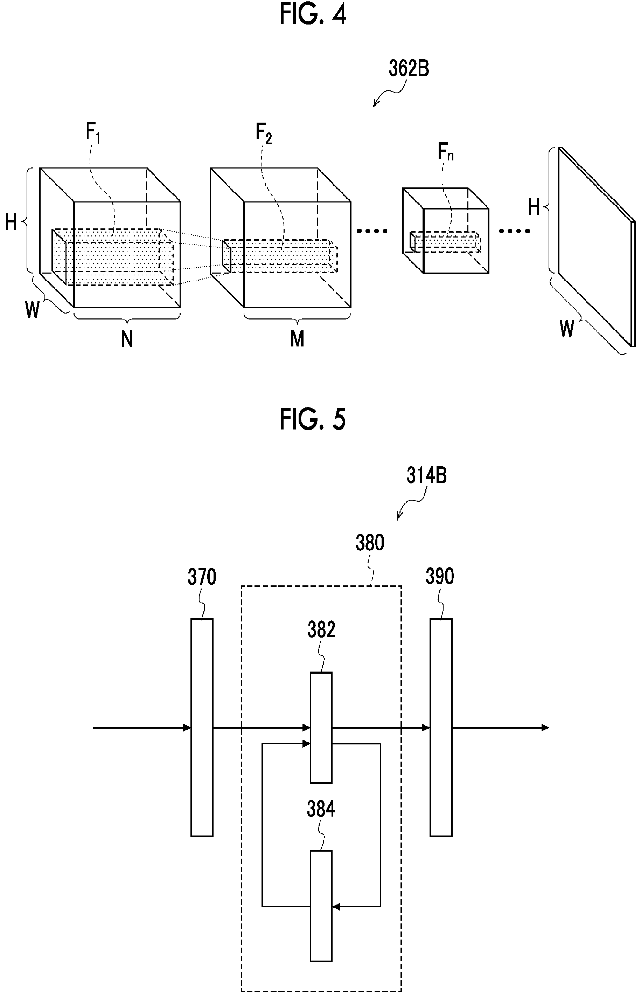

Fig. 4 is a schematic diagram showing a configuration example of the interlayer 362B of the recognizer 314A shown in Figs. 3A and 3B. In the first convolutional layer of the interlayer 362B, the convolution operation between an image set (learning image set during learning, recognition image set during recognition) composed of a plurality of medical images and a filter F₁ is performed. The image set is composed of N (N channels) images having an image size of H in a vertical direction and W in a horizontal direction. In a case where a color image is input, the images constituting the image set are three-channel images of red (R), green (G), and blue (B), but the number of channels varies depending on the modality such as a CT image or an MRI image. The filter F₁ used in the convolution operation with this image set is, for example, a filter having a filter size of 5 x 5 x N in a case of a filter having a size 5 (5 x 5) because the image set has N channels (N images). Through the convolution operation using the filter F₁, a "feature map" of one channel (one image) is generated for one filter F₁. A filter F₂ used in the second convolutional layer has, for example, a filter size of 3 x 3 x M in a case of a filter having a size 3 (3 x 3).

Similar to the first convolutional layer, in the second to n-th convolutional layers, convolution operations using filters F₂ to Fₙ are performed. The reason why the size of the "feature map" in the n-th convolutional layer is smaller than the size of the "feature map" in the second convolutional layer is that the feature map is downscaled by the convolutional layer or the pooling layer up to the preceding stage.

Among the layers of the interlayer 362B, lower-order feature extraction (edge extraction or the like) is performed in the convolutional layer closer to the input side, and higher-order feature extraction (extraction of features related to the shape, structure, and the like of the object) is performed as it approaches the output side. In a case where segmentation is performed for the purpose of measurement or the like, the convolutional layer in the latter half is upscaled, and in the last convolutional layer, a "feature map" of the same size as the input image set is obtained. On the other hand, in the case of performing object detection, upscaling is not essential because position information is only required to be output.

The interlayer 362B may include a layer for batch normalization in addition to the convolutional layer 364 and the pooling layer 365. The batch normalization process is processing of normalizing the distribution of data in units of mini-batch during learning and plays a role of advancing learning quickly, reducing dependence on initial values, suppressing overtraining, and the like.

### Processing in output layer

The output layer 362C is a layer that performs the position detection of the region of interest appearing in the input medical image based on the feature amount output from the interlayer 362B and that outputs the result. In a case where segmentation is performed, the output layer 362C grasps the position of the region of interest shown in the image at the pixel level by the "feature map" obtained from the interlayer 362B. That is, it is possible to detect whether or not each pixel of the medical image belongs to the region of interest and output the detection result. On the other hand, in a case where object detection is performed, the determination in the pixel level is not necessary, and the output layer 362C outputs the position information of the object.

The output layer 362C may execute the discrimination (classification) regarding the region of interest and output the discrimination result. For example, the output layer 362C classifies medical images into three categories of "neoplastic", "non-neoplastic", and "other", and may output a discrimination result as three scores (the sum of the three scores is 100%) corresponding to "neoplastic", "non-neoplastic" and "other" or may output a classification result in a case where it can be clearly classified from the three scores. In a case of outputting the discrimination result, the output layer 362C may or may not include the fully-connected layer as the last one layer or a plurality of layers (refer to Fig. 3B).

The output layer 362C may output a measurement result of the region of interest. In a case where measurement is performed by the CNN, the target region of interest can be segmented, for example, as described above, and then can be measured by the processor 310 based on the result. In addition, the measurement value of the target region-of-attention can also be directly output from the recognizer 314A. In a case where the measurement value is directly output, it is a regression problem of the measurement value because the measurement value itself is trained for the image.

In a case in which the CNN having the above-described configuration is used, it is preferable to, in the process of the learning, perform processing (error back propagation) of comparing a result output by the output layer 362C with a correct answer of the recognition for the image set to calculate a loss (error), and updating the weight parameter in the interlayer 362B from the output-side layer toward the input-side layer so that the loss is reduced.

The recognizer applied to the medical image (for example, the thin-slice CT image, and the high-resolution image and the material discrimination image by the PCCT device 100) stored in the transitory storage region 412 may be a computer aided diagnosis/detection (CAD) that uses another method of machine learning (artificial intelligence) instead of a recognizer using a neural network, such as the recognizer 314A.

The processor 310 may comprise a plurality of recognizers corresponding to the features of the organ, the part, and the region of interest. The recognizers may be, for example, recognizers for adrenal gland tumor, urinary stone, gallstone, liver fat amount, and the like. In addition, the recognizer may have different algorithms (machine learning methods) or parameters depending on the features of the organ, the part, or the region of interest.

### Configuration of detector

Fig. 5 is a schematic diagram showing a configuration example of the detector 314B by an RNN. The detector 314B has an input layer 370, a hidden layer 380, and an output layer 390, and is different from a typical neural network in that the hidden layer 380 has a hidden layer 382 indicating a state at the current time point (time point t) and a hidden layer 384 indicating a state at the past time point (time point t-1). The detector 314B can perform estimation using a past history (in the present embodiment, a context of characters, words, and phrases in medical comments on findings) of information that is input in a time series manner, such as a natural language, by holding a state of the hidden layer at the time point t-1 and using the state for input at the next time point t. The detector 314B may include a plurality of configurations of Fig. 5 or may be hierarchically configured. The detector 314B may be configured using long short-term memory (LSTM), which is a type of RNN.

The detector 314B detects specific information from the medical comments on findings using a natural language processing method. In addition, the detector 314B can set a specific phrase as the detection target as the "specific information", but the "specific phrase" may be a phrase related to the region of interest. The phrase related to the region of interest may be, for example, one or more of the presence or absence, position, type, state or degree, number, shape, or dimension of the region of interest. In addition, the "specific phrase" may be information indicating an organ that is a target of the medical comments on findings, such as the name of the organ in which the region of interest has been detected. The user of the medical information processing system 10 can set what information or what phrase is to be a detection target.

### Storage device

The storage device 340 (output device) is configured with a non-transitory recording medium such as a magneto-optical recording medium and various semiconductor memories, and a controller thereof, and can store medical data including at least one of a medical image or medical comments on findings related to the medical image, a detection condition of medical information (what information or phrase is detected, a detection method, a detection timing, and the like), a detection result of the medical information, information indicating a history of movement, and the like.

### Display device and operating device

The information processing device 300 comprises a display device (one aspect of an output device) and an operating device (keyboard, mouse, or the like) (which are not shown). The user of the information processing device 300 can input an instruction necessary for operating the information processing device 300 via these devices. The information stored in the storage device 340 and the execution result of the operation method of an information processing device according to the embodiment of the present invention can be displayed on the display device in response to the instruction of the user through the display device and the operating device or automatically regardless of the instruction of the user.

In addition to the above-described components, the information processing device 300 comprises the communication interface 350 for communicating with other devices of the medical information processing system 10 via the network 20.

### Report server, viewer server, and viewer terminal

The viewer terminal 600 receives an operation of user related to viewing of a medical image, input of medical comments on findings, and viewing of a report via an operating device (keyboard, mouse, microphone, and the like) (not shown), acquires necessary information by communicating with the viewer server 500 and the like as necessary, and displays the information on a display device (liquid crystal display device and the like) (not shown). The viewer server 500 transmits and receives necessary information to and from the PACS 400, the information processing device 300, and the report server 700. The report server 700 manages the storage and viewing of the report created by the viewer terminal 600. The report server 700 may store the created report in a storage device of the report server 700 or in the storage device 410 of the PACS 400. The report server 700 can be omitted, and in this case, the functions of the report server 700 can be implemented by other devices such as the PACS 400.

### Processing in medical information processing system (first aspect)

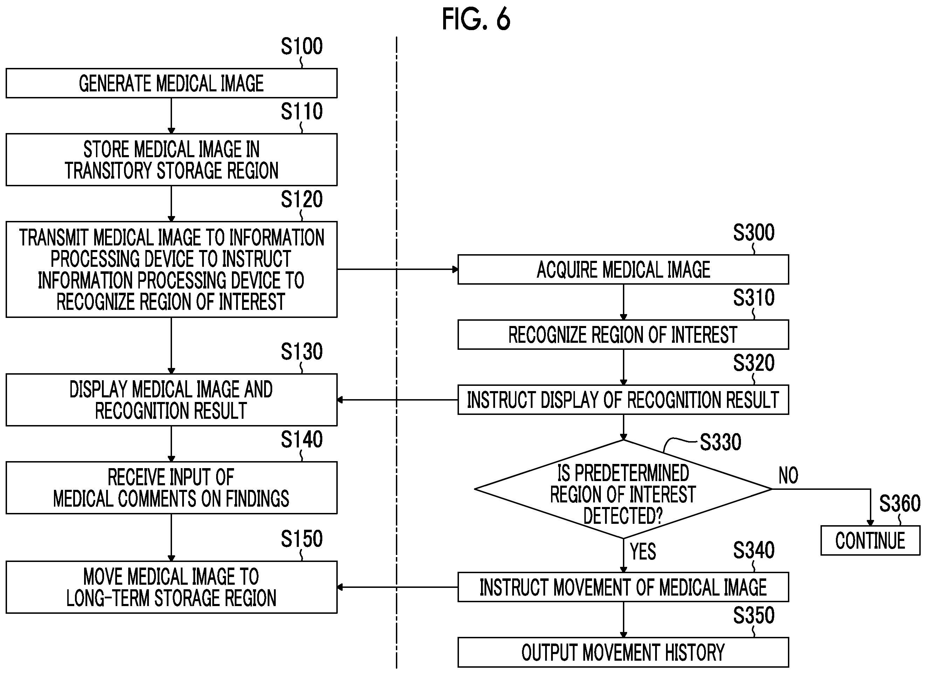

Next, processing in the medical information processing system 10 having the above configuration will be described. Fig. 6 is a flowchart showing processing in the medical information processing system 10. In Figs. 6, 8, and 9, steps on the right side of a one-dot chain line in the drawing indicate processing in the information processing device 300, and steps on the left side of the one-dot chain line indicate processing in devices other than the information processing device 300. In addition, the order of processing in these figures is an example, and may be changed as necessary.

### Generation of medical image

A medical image capturing device such as the PCCT device 100 generates a medical image of the subject in response to an instruction from the user (step S100). For example, the PCCT device 100 can generate a normal CT image, but may generate one type of CT image (one aspect of the first medical image) or a plurality of CT images (one aspect of the first and second medical images) having different imaging parameters and/or contents of image processing. In addition, the PCCT device 100 may generate a normal CT image and a material discrimination image (one aspect of the first and second medical images), or may generate a plurality of types of material discrimination images.

In addition, the medical image may be generated by another modality (the MRI device 200 in the aspect of Fig. 1) instead of or in addition to the PCCT device 100. In a case where the medical information processing system 10 includes other medical image capturing devices such as a normal CT device or an ultrasound imaging device, a medical image may be generated by those devices. Medical images by these modalities are also one aspect of the first and second medical images in the embodiment of the present invention.

As described above, in the medical information processing system 10 according to the first embodiment, a plurality of medical images can be generated. These medical images are one aspect of "a first medical image and a second medical image which is related to the first medical image and is different from the first medical image in a type of image". In the first embodiment, as an example of "the first medical image and the second medical image are related to each other", it is possible to mention that the subjects are the same, the imaging dates and times are the same or close to each other, the imaging parts or slice positions are common, and the like. In addition, regarding "be different in a type of image", the first medical image and the second medical image may be different from each other in at least one of a modality, an imaging parameter, or a content of image processing.

### Storage of medical image in transitory storage region

The PACS 400 stores the medical image acquired in step S100 in the transitory storage region 412 (step S110), and transmits the medical data including the medical image to the information processing device 300 to instruct the information processing device 300 to recognize the region of interest (step S120). The region of interest may be at least one of a lesion, a lesion candidate region, or a treated region.

In the present invention, the "medical data" includes at least one of the medical image or the medical comments on findings related to the medical image. In the following first aspect, a case where the medical data includes only the medical image out of the medical image and the medical comments on findings will be described, and a case where the medical data includes the medical image and the medical comments on findings (second aspect) will be described later.

### Acquisition of medical image and recognition of region of interest

The processor 310 (processor) of the information processing device 300 acquires a medical image (medical data) (step S300), and applies a machine learning method to the acquired medical image to recognize a region of interest (step S310). Specifically, the recognizer 314A configured by the neural network as described above detects the region of interest from the medical image. Here, it is assumed that the detection is performed as the "recognition" of the region of interest, but the recognizer 314A may perform discrimination or measurement. In a case where the recognition is performed, the processor 310 transmits the recognition result to the viewer server 500 (and/or the report server 700), and instructs the display device of the viewer terminal 600 to display the medical image and the recognition result (step S320). The viewer server 500 displays the medical image and the recognition result on the display device of the viewer terminal 600 in response to this instruction (step S130).

### Identifiable display/highlight of region of interest and notification of detection

In a case of displaying the medical image and the recognition result, the viewer server 500 may identifiably display and/or highlight the detected region of interest, or may notify that the region of interest has been detected, according to the instruction of the processor 310 in response to the operation of the user. The aspect of the identifiable display and/or the highlight includes, for example, display of characters, numbers, figures, symbols, and the like, and colored display of the region of interest, and similarly, the aspect of the notification includes, for example, display of characters, numbers, figures, symbols, and the like, and output of a voice. The processor 310 can determine the aspects of the identifiable display, the highlight, and the notification in response to an instruction of the user via the operator of the information processing device 300 or the operator of the viewer terminal 600.

The viewer terminal 600 receives the input of the medical comments on findings for the medical image according to the operation of the user (step S140), and associates the medical image with the medical comments on findings according to the input. The association is performed by, for example, tag information of image data in Digital Imaging and Communications in Medicine (DICOM) format, but other formats may be used. The report server 700 stores the associated medical image and medical comments on findings in a storage device (not shown). In a case where the medical information processing system 10 does not comprise the report server 700, the PACS 400 may store the associated data in the storage device 410.

### Movement of medical image to long-term storage region

The recognizer 314A (processor 310) determines whether or not a predetermined region of interest is detected from the medical image based on the result of step S310 (step S330). The "predetermined region of interest" is one aspect of the "predetermined medical information", and may be, for example, a region of interest having a specific feature (type, shape, size, quantity, degree, and the like). The "what region of interest is detected" is recorded in the storage device 340 or the like, and the processor 310 can perform the determination with reference to the recorded content.

In a case where the predetermined region of interest is detected (YES in step S330), the movement controller 316 (processor) instructs the PACS 400 to move the medical image to the long-term storage region 414 (step S340). In addition, the movement controller 316 (processor) outputs information indicating the history of movement to the output device (step S350). This "output device" may be the display device (not shown) or the storage device 340 of the information processing device 300, the display device of the viewer terminal 600, the storage device 410 of the PACS 400, or the like. The movement controller 316 may notify the user of the movement of the medical image in a case of the movement (screen display, voice output, or the like).

In addition, the PACS 400 moves the medical image stored in the transitory storage region 412 to the long-term storage region 414 in response to an instruction (step S340) from the processor 310 (information processing device 300). Therefore, the PACS 400 may output information indicating the history of movement to an output device (the storage device 410 or a display device (not shown)).

### Movement of first and second medical images

As described above, in the medical information processing system 10, a plurality of types of medical images can be generated by the PCCT device 100 and the MRI device 200. Therefore, in a case where the plurality of types of medical images are generated in step S100, the movement controller 316 (processor) and the PACS 400 may move the medical images to the long-term storage region 414.

Specifically, in a case where the first medical image and the second medical image which is related to the first medical image and which is different from the first medical image in a type of image are generated (for example, a normal CT image and a material discrimination image, a CT image and an MRI image, and the like), the first medical image and the second medical image may be moved to the long-term storage region 414 in a case where a predetermined region of interest is detected in the first medical image. In addition, in this case, the medical image in which the designated organ and/or region of interest is shown out of the second medical image may be moved to the long-term storage region. Thereby, the data size can be reduced.

In step S130, the medical image is displayed on the display device of the viewer terminal 600 together with the recognition result (result of detection), but this display may or may not be used as a requirement for moving the medical image.

In a case where the predetermined region of interest is not detected in step S330, the medical information processing system 10 continues the processing without moving the medical image (step S360).

### Information on movement history

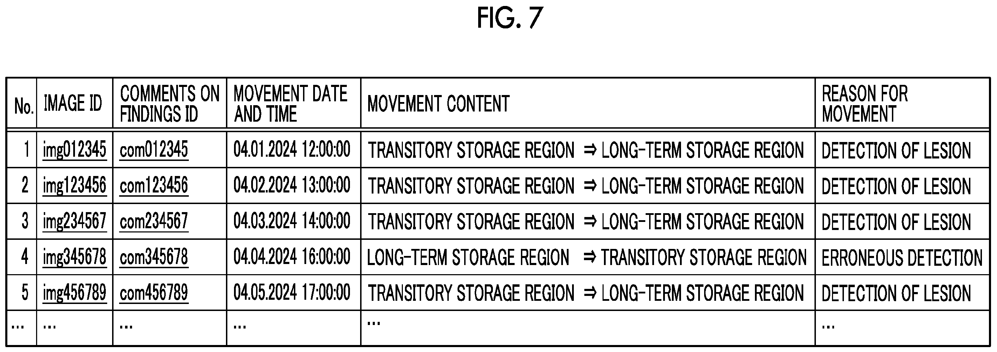

Fig. 7 is a diagram showing an output example of information on a history of movement. As described above, the output may be performed by a storage device or a display device. In the example of Fig. 7, the IDs of the medical image and the medical comments on findings, the movement date and time, the movement content, and the reason for the movement are summarized in a table format, and the user can easily grasp the movement history by viewing this table. The medical image and the medical comments on findings are linked, and the user can view the medical image and the medical comments on findings by designating the ID. The movement controller 316 (processor) and/or the PACS 400 may output (display or record) information indicating the medical image stored in the transitory storage region 412 and/or information indicating the medical image stored in the long-term storage region 414 to the output device, instead of the information indicating the history of movement.

### Processing in case of erroneous detection

As described above, in the present embodiment, in a case where the determined region of interest is detected from the medical image, the medical image is moved to the long-term storage region 414, but this detection may be erroneous. In such a case, since it is not necessary to move the medical image to the long-term storage region 414, the medical image can be restored to the transitory storage region 412 as follows.

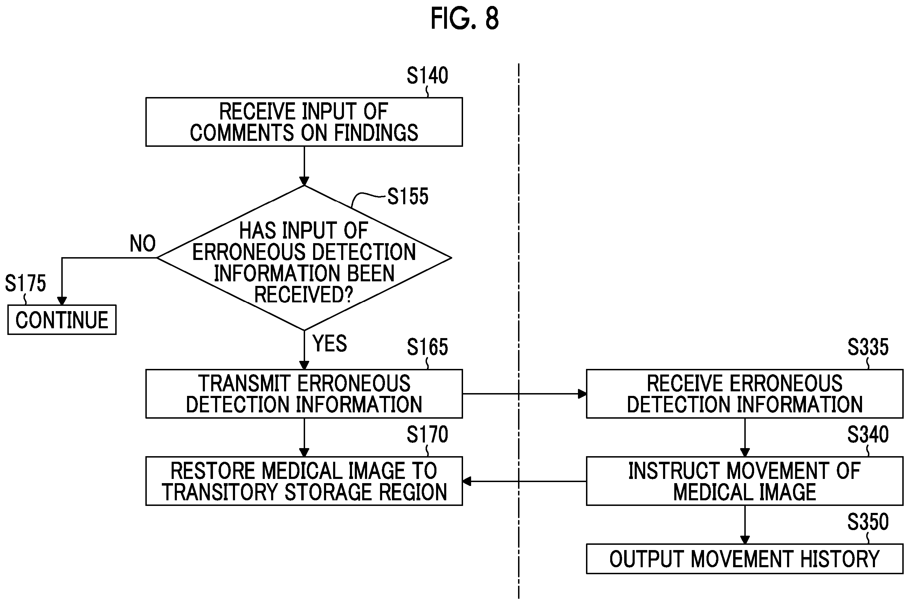

Fig. 8 is a flowchart showing processing in a case of erroneous detection (processing of restoring a medical image moved to the long-term storage region to the transitory storage region). The viewer server 500 determines whether or not the viewer terminal 600 has received the input of the information indicating the erroneous detection (step S155). In a case where the user notices the erroneous detection by interpreting the medical image (for example, in a case where the user notices that the detection result is false positive (FP)), the user can operate the viewer terminal 600 to input information indicating the erroneous detection. The user can input this information by performing a determined operation such as clicking a predetermined button on a screen of a display device or selecting a menu for erroneous detection notification.

In addition, the user may input the "information indicating erroneous detection" as a part of the medical comments on findings. For example, it is conceivable to input a phrase such as "erroneous detection" or "the presence of the region of interest is not recognized" into the medical comments on findings. In this case, the viewer server 500, the report server 700, or the like can detect such a phrase from the medical comments on findings and perform the determination in step S155 based on the detection result. The viewer server 500 or the like may perform such detection based on a machine learning method such as natural language processing. In addition, the viewer server 500 or the like may transmit the medical comments on findings to the information processing device 300, and the information processing device 300 may perform the determination as in step S155.

In a case where the viewer server 500 receives the input of the information indicating the erroneous detection (YES in step S155), the viewer server 500 transmits the information to the information processing device 300 (step S165), and the information processing device 300 receives the information (step S335). The movement controller 316 (processor) instructs the PACS 400 to restore the medical image moved to the long-term storage region 414 to the transitory storage region 412 in response to the reception (acquisition) of the information (step S340), and the PACS 400 restores the medical image to the transitory storage region 412 in response to this instruction (step S170).

In addition, the movement controller 316 outputs information indicating the movement history to the output device in this case (step S350). The movement controller 316 may notify the user of the movement of the medical image in a case of the movement (screen display, voice output, or the like).

In the processing in a case of the erroneous detection, in a case where all the detection results of the series of medical images (for example, the medical images acquired in one examination) are erroneous detection (FP or the like), the medical images may be restored to the transitory storage region 412. In addition, in the determination of the erroneous detection, instead of the user inputting the information as described above, the information processing device 300 or the like may perform image processing on the medical image or apply the recognizer to the medical image to collate the medical image with the recognition result in step S310 and determine whether or not the erroneous detection is performed. This recognizer (recognizer for erroneous detection determination) can also be constructed by a machine learning method, and may be different from the recognizer 314A in terms of an algorithm, a layer configuration, a parameter, and the like.

As described above, with the medical information processing system 10 according to the first embodiment, in a case where the predetermined medical information is detected from the medical data, the medical image stored in the transitory storage region 412 is automatically moved to the long-term storage region 414. Therefore, it is possible to reduce a possibility of a situation in which "the medical image stored in the transitory storage region has been deleted, and thus the image cannot be referred to even though the image is desired to be referred to", and it is possible to appropriately store the medical image. In addition, even for a medical image for which the user has not registered as the key image or performed bookmarking, in a case where the predetermined medical information is detected, the medical image can be moved to the long-term storage region 414, and it is possible to reduce a burden on the user regarding the storage of the medical image.

### Processing in medical information processing system (second aspect)

Next, a second aspect of processing in the medical information processing system 10 will be described. In the first aspect described above, the case where the medical data includes only the medical image out of the medical image and the medical comments on findings has been described, but in the second aspect, the medical data includes the medical image and the medical comments on findings.

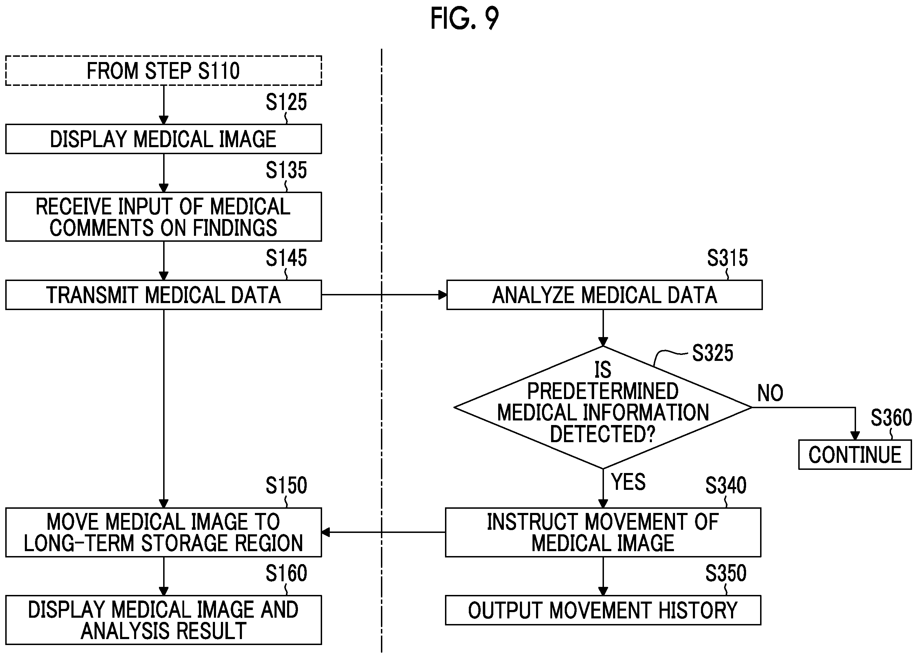

Fig. 9 is a flowchart showing processing in the second aspect. The same step number is assigned to the same processing as the processing in the first aspect (Fig. 6), and detailed description thereof is omitted.

The viewer server 500 displays the medical image on the display device of the viewer terminal 600 in response to an instruction from the processor 310 (information processing device 300) (step S125). In a case where the user interprets the displayed medical image and inputs the medical comments on findings by operating the viewer terminal 600, the viewer terminal 600 receives the input (step S135). The viewer server 500 transmits the medical data including at least one of the medical image or the medical comments on findings to the information processing device 300 (step S145), and the information processing device 300 analyzes the received (acquired) medical data (step S315).

The medical information detection unit 314 (processor) can perform at least one of detecting a determined region of interest from the medical image by the recognizer 314A or detecting specific information from the medical comments on findings by the detector 314B as the "analysis". In this "analysis", the detection of the determined region of interest or the detection of the specific information is one aspect of "detection of predetermined medical information" in the present invention.

In a case where only one of the medical image or the medical comments on findings is received from the viewer server 500, the medical information detection unit 314 can perform one processing corresponding to the received information, and in a case where both the medical image and the medical comments on findings are received, the medical information detection unit 314 can perform one processing or both processing. In a case where the medical information detection unit 314 performs both processing, the movement controller 316 can determine to move the medical image in a case where the detection results match. The medical information detection unit 314 may determine which processing is performed according to the operation of the user, or may automatically determine which processing is performed regardless of the operation of the user. As described above, these types of processing can be executed using a machine learning method (including a natural language processing method). The "specific information" may be information indicating a specific phrase or a specific organ, and the "specific phrase" may be a phrase related to a determined region of interest.

In a case where a specific phrase is detected, the movement controller 316 can move a medical image related to the phrase. For example, in a case where "iodine density" and "fat" are detected as the specific phrase, the movement controller 316 can move the iodine density image and the fat density image, respectively. In addition, the movement controller 316 may determine the organ in which the findings are described based on the detection result of the information indicating the specific phrase or the specific organ, and may move only the slice in which the organ is shown to the long-term storage region 414.

The "specific information" may be plural than one, and in a case where a plurality of pieces of information are detected, it is preferable to consider the relevance of the pieces of information. For example, it is preferable that the movement controller 316 determines whether or not movement is necessary in consideration of the relevance of a plurality of detected phrases (in this case, "tumor", "recognition", "non-recognition", and the like), such as "recognition of the presence of the tumor" and "non-recognition of the presence of the tumor", in addition to only the phrase "tumor".

In a case where the predetermined medical information is detected from the medical data (YES in step S325), the movement controller 316 (processor) instructs the PACS 400 to move the medical image stored in the transitory storage region 412 to the long-term storage region 414 (step S340). The PACS 400 moves the medical image to the long-term storage region 414 in response to this instruction (step S150), and displays the medical image and the analysis result thereof (the analysis result in step S315) on the display device of the viewer terminal 600 (step S160). In addition, the movement controller 316 (processor) outputs information indicating the history of movement (see the example in Fig. 7) to the output device (step S350). In a case where the predetermined medical information is not detected from the medical data (NO in step S325), the processor 310 (information processing device 300) does not issue an instruction of movement of the medical image and continues the processing (proceeds to step S360).

The movement controller 316 (processor) may perform the determination in step S325 based on one of the detection result from the medical image or the detection result from the medical comments on findings, or may perform the determination based on both.

In the second aspect, as in the first aspect, it is possible to respond to the erroneous detection of the medical information. The user can input information indicating the erroneous detection or apply the recognizer to the medical image to detect the determined region of interest, allowing the processor 310 to acquire the information indicating the erroneous detection from the result thereof.

As described above, even in the second aspect, it is possible to appropriately store the medical image as in the first aspect, and it is possible to reduce the burden on the user regarding the storage of the medical image.

Hereinbefore, the embodiment of the present invention has been described above, but the present invention is not limited to the above-described aspects, and various modifications can be made.

### Explanation of References

10: medical information processing system
20: network
100: PCCT device
200: MRI device
300: information processing device
310: processor
312: input/output controller
314: medical information detection unit
314A: recognizer
314B: detector
316: movement controller
340: storage device
350: communication interface
362A: input layer
362B: interlayer
362C: output layer
364: convolutional layer
365: pooling layer
366: fully-connected layer
370: input layer
380: hidden layer
382: hidden layer
384: hidden layer
390: output layer
410: storage device
412: transitory storage region
414: long-term storage region
500: viewer server
600: viewer terminal
700: report server

## Claims

1. An information processing device comprising a processor,
wherein the processor is configured to:
acquire medical data including at least one of a medical image or medical comments on findings related to the medical image;
apply a machine learning method to the medical data to detect predetermined medical information from the medical data; and
move the medical image stored in a transitory storage region of a storage device to a long-term storage region of the storage device in a case where the medical information is detected.

2. The information processing device according to claim 1,
wherein the processor is configured to:
restore the medical image moved to the long-term storage region to the transitory storage region in a case where the processor acquires information indicating that a result of the detection is erroneous detection.

3. The information processing device according to claim 1 or 2,
wherein the processor is configured to:
move the medical image from the transitory storage region to the long-term storage region in a case where the processor acquires information indicating that the medical image is displayed on a display device together with a result of the detection.

4. The information processing device according to any one of claims 1 to 3,
wherein the processor is configured to:
move a medical image related to specific information from the transitory storage region to the long-term storage region in a case where the specific information is detected from the medical comments on findings.

5. The information processing device according to claim 4,
wherein the processor is configured to:
detect information indicating an organ which is a target of the medical comments on findings as the specific information from the medical comments on findings and move a medical image in which the organ is shown from the transitory storage region to the long-term storage region.

6. The information processing device according to claim 4 or 5,
wherein the processor is configured to:
detect a specific phrase as the specific information from the medical comments on findings and move a medical image related to the specific phrase, and
preferably, the processor is configured to:
detect a phrase related to a region of interest as the specific phrase.

7. The information processing device according to any one of claims 4 to 6,
wherein the processor is configured to:
detect the specific information from the medical comments on findings using a natural language processing method.

8. The information processing device according to any one of claims 1 to 7,
wherein the processor is configured to:
move a first medical image and a second medical image which is related to the first medical image and is different from the first medical image in a type of image, from the transitory storage region to the long-term storage region, in a case where a region of interest is detected in the first medical image, and
preferably, the second medical image is an image different from the first medical image in at least one of a modality, an imaging parameter, or a content of image processing.

9. The information processing device according to claim 8,
wherein the processor is configured to:
move a medical image in which a designated organ and/or region of interest is shown, out of the second medical image.

10. The information processing device according to any one of claims 1 to 9,
wherein the processor is configured to:
acquire a CT image and/or a material discrimination image as the medical image, and
preferably, the CT image and the material discrimination image are images captured by a photon counting CT device.

11. The information processing device according to any one of claims 6, 8 and 9,
wherein the region of interest is at least one of a lesion, a lesion candidate region, or a treated region.

12. The information processing device according to any one of claims 1 to 11,
wherein the processor is configured to:
cause an output device to output information indicating the medical data stored in the transitory storage region and/or information indicating the medical data stored in the long-term storage region.

13. The information processing device according to any one of claims 1 to 12,
wherein the processor is configured to:
cause an output device to output information indicating a history of movement of the medical image between the transitory storage region and the long-term storage region.

14. The information processing device according to any one of claims 1 to 13,
wherein the processor is configured to:
move the medical image to a region that stores the medical image for a longer period of time than the transitory storage region by using the region as the long-term storage region.

15. An operation method of an information processing device including a processor, the method comprising:
via the processor,
acquiring medical data including at least one of a medical image or medical comments on findings related to the medical image;
applying a machine learning method to the medical data to detect predetermined medical information from the medical data; and
moving the medical image stored in a transitory storage region of a storage device to a long-term storage region of the storage device in a case where the medical information is detected.
